# EUROPEAN PATENT SPECIFICATION

(11) **EP 3 057 540 B1**
(45) Date of publication and mention of the grant of the patent: **27.11.2019**
(21) Application number: 14789707.8
(22) Date of filing: 09.10.2014
(51) Int. Cl.: A61F 2/915, A61F 2/82

(54) **VASCULAR STENT**
VASKULÄRER STENT
ENDOPROTHÈSE VASCULAIRE

(30) Priority: 16.10.2013 US 201314055120
(43) Date of publication of application: 24.08.2016
(73) Proprietor: Covidien LP, Mansfield, MA 02048 (US)
(72) Inventor: NOFFKE, Paul, St. Paul, MN 55117 (US); VOGEL, Jeffrey H., Brooklyn Park, MN 55443 (US); SCHANTELL, Robert E., Plymouth, MN 55442 (US)
(74) Representative: Gray, James
(86) International application number: PCT/US2014/059799
(87) International publication number: WO 2015/057469

(56) References cited:
- WO-A1-97/25937
- WO-A2-2012/096716
- US-A1- 2007 100 434

## Description

### BACKGROUND

### 1. Technical Field

The present disclosure generally relates to luminal implants, and, in particular, relates to a vascular stent having improved performance characteristics to facilitate deployment and implantation within the venous system.

### 2. Description of Related Art

Stents are widely used for numerous medical applications where the stent is placed in the lumen of a subject and expanded. Stents may be used in the coronary or the peripheral vasculature, as well as other body lumens. Typically, stents are metal, tubular structures which are passed through a body lumen in a collapsed state. At the point of an obstruction or other deployment site in the body lumen, the stent is expanded to support the lumen. Stents may be self-expanding or balloon-expandable. Self-expanding stents are generally inserted in a constrained state into vasculature via a delivery device and released whereby the unconstrained stent is free to radially expand. A balloon-expandable stent is positioned on a balloon of a balloon catheter. The stent is expanded at the site through inflation of the balloon.

Lateral and radial strength, fracture resistance and uniform strain distribution are desirable characteristics of a stent. These characteristics must be addressed in stent design without sacrificing stent flexibility in both the longitudinal and radial directions. Stent flexibility is paramount when the stent is positioned within a subject's vasculature at or near a subject's joint (e.g., hip, pelvis, knee, elbow, etc.). In these regions, the stent is subjected to torsion, bending and other mechanical stress. Moreover, stents for use in the venous system such as inferior vena cava (IVC), common iliac, external iliac, and common femoral veins regions require high strength and maximum flexibility.

Published PCT application WO 2012/096716 A2 relates to a medical device and a method of using it. The device is a stent which can be percutaneously deliverable with (or on) an endovascular catheter or via other surgical or other techniques and then expanded. The stent is configured to have a central portion defined by "open" cells and at least two end portions, defined by "closed" cells, spaced apart and directly connected to the distal and proximal ends of the central portion of the stent. The stent may also optionally have a covering or a lattice with openings.

Published U.S. Patent application 2007/100434 A1 describes a stent that may comprise a plurality of serpentine bands, wherein adjacent serpentine bands are connected by at least one connector strut. A serpentine band may comprise alternating straight struts and s-shaped struts, wherein connector struts may comprise first connector struts and second connector struts, and wherein the first connector struts are nonparallel to the second connector struts.

### SUMMARY

Accordingly, the present disclosure is directed to further improvements in stents, particularly, vascular stents. In accordance with an embodiment, there is provided a medical stent comprising:
a stent body defining a longitudinal axis "K" and opposed longitudinal ends, the stent body adapted to expand from an initial condition to an expanded condition, the stent body including a plurality of longitudinal cells, the longitudinal cells including a first end cell and a second end cell opposite each other and at least one intermediate cell disposed between the first end cell and the second end cell;
each longitudinal cell having first and second structural members extending in an undulating pattern about the longitudinal axis "K";
x number of intermediate connectors interconnecting the first and second structural members of the at least one intermediate cell; and
at least 2x the number of end connectors interconnect the first and second structural members of each of the first end cell, and the second end cell,
wherein the end connectors comprise a first end connector and a second end connector interconnecting the first and second structural members of the first end cell and the second end cell, and
wherein in the initial condition of the stent body, the first end connector is arranged at a positive first oblique angle relative to the longitudinal axis "K", and the second end connector is arranged at a negative second oblique angle relative to the longitudinal axis "K".

In embodiments, the intermediate connectors each may be arranged in general parallel relation with the longitudinal axis.

Cell connectors may interconnect adjacent longitudinal cells. In embodiments, the number of cell connectors interconnecting each end cell to the at least one intermediate cell is equal to the number of intermediate connectors of each intermediate cell.

In some embodiments, the stent body may include at least two of the intermediate cells, each interconnected by cell connectors.

In further embodiments, the first and second structural members each include a plurality of struts with adjacent struts being interconnected by a node. The end and intermediate connectors may be dimensioned to interconnect longitudinally adjacent nodes of the first and second structural members of the end cell and the at least one intermediate cell respectively. At least some of the longitudinally adjacent nodes of the at least one intermediate cell are circumferentially offset relative to each other when in the expanded condition of the stent body.

In some embodiments, the nodes include first and second node types with the first node type having an internal curvature defining at least a first radius of curvature, and the second node type having an internal curvature defining either no curvature or a second radius of curvature less than the first radius of curvature.

A process for forming a stent may include:
forming a stent pattern in a tubular member comprising a shape memory material, the stent pattern including longitudinal cells having opposed end cells and at least one intermediate cell disposed between the end cells, each cell having a plurality of undulating struts with adjacent struts being interconnected by a node;
positioning the tubular member on a mandrel, the mandrel including a cylindrical member and having a series of projections extending radially outwardly from an outer wall of the tubular member;
rotating at least one end of the tubular member about the central axis to circumferentially displace at least some of the nodes thereby assuming a circumferentially displaced condition thereof;
arranging respective individual nodes of the longitudinal cells onto the series of projections of the mandrel to maintain the nodes in the circumferentially displaced position; and
subjecting the tubular member when on the mandrel to heat to heat set the tubular member with the nodes in the radially displaced condition.

A mandrel for use in manufacturing a stent may include a cylindrical member defining an outer wall and a central axis therethrough, and a series of projections extending radially outwardly from the outer wall. Each series of projections is arranged in a helical pattern about the outer wall and relative to the central axis, each projection having opposed sides which converge and terminate at an apex.

Embodiments of the present disclosure may include one or more of the following advantages.

The configuration of the stent body including the end cells and the intermediate cells provide substantial advantages when placed in, e.g., the venous system of the subject. The increased number of the end connectors within the end cells provides stability and radial strength to the longitudinal ends of the stent body. This increased stability advantageously prevents jumping or premature "flowering" of the stent during deployment. The relative reduced number of intermediate connectors within the intermediate cells provides the requisite flexibility required for implantation within the venous site, such as the ileofemoral veins, and accommodates for movement of the subject.

Other aspects, features, and advantages will be apparent from the description, drawings, and the claims.

### BRIEF DESCRIPTION OF THE DRAWINGS

Embodiments of the present disclosure will be readily appreciated by reference to the drawings wherein:
FIG. 1 is a side elevation view of a stent of the present disclosure in an initial unexpanded condition;
FIG. 2 is an enlarged view of the area of isolation depicted in FIG. 1 illustrating an end cell of the stent;
FIG. 3 is an enlarged view of an enclosed cell segment of the end cell;
FIG. 4 is a enlarged view of the area of isolation depicted in FIG. 1 illustrating an intermediate cell of the stent;
FIG. 5 is an enlarged view of the area of isolation depicted in FIG. 1;
FIG. 6 is a flow chart depicting a method of manufacture of the stent;
FIG. 7 is a perspective view of a mandrel utilized in the method of manufacture;
FIG. 8 is a plan view of the mandrel of FIG. 7 unrolled and laid flat;
FIG. 9 is an enlarged view of the area of isolation depicted in FIG. 8;
FIGS. 10-11 are views illustrating loading of the stent on the mandrel;
FIG. 12 is a view illustrating the stent fully loaded on the mandrel;
FIG. 13 is a plan view of the stent unrolled and laid flat and in an expanded condition; and
FIG. 14 is an enlarged view of the area of detail depicted in FIG. 13.

### DESCRIPTION

The stent of the present disclosure has particular application in the vasculature of a subject where it is subjected to a relatively high amount of strain and movement. For example, the stent may be suitable for use within the vasculature of a subject's hip area, e.g., to help reduce problems associated with a deep vein thrombosis (DVT). The stent may be placed in the inferior vena cava (IVC), common iliac, external iliac, and common femoral veins for chronic venous obstructions and/or May-Thurner syndrome. Although the stent has particular application in the venous system, the stent may be used in the coronary artery, peripheral arteries and in the neurovasculature. The stent also may have application in the upper and lower gastrointestinal tract. The stent may be a component of an apparatus or system used in conjunction with any of the above applications.

The various examples of the disclosure will now be described in connection with the drawings. It should be understood that for purposes of better describing the disclosure, the drawings may not be to scale. Further, some of the figures may include enlarged or distorted portions for the purpose of showing features that would not otherwise be apparent.

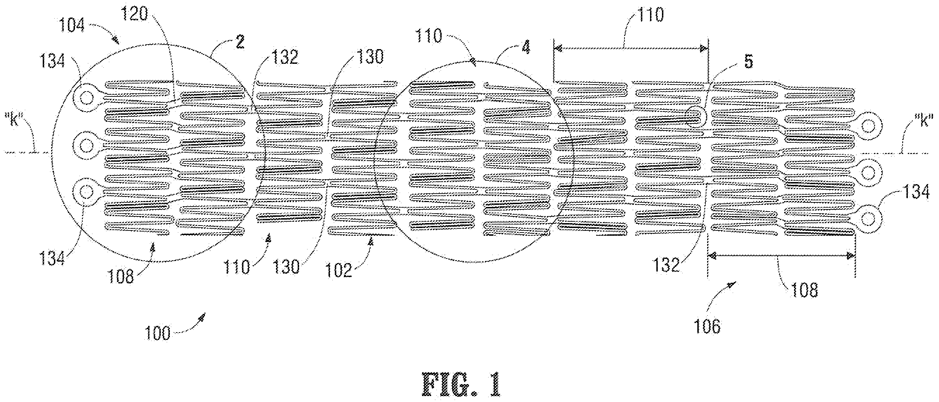

With initial reference to FIG. 1, the stent 100 of the present disclosure is illustrated. The stent 100 includes a stent body 102 defining a central longitudinal axis "k" extending the length of the stent body 102 and opposed longitudinal ends 104, 106. The stent body 102 includes a plurality of longitudinal cells. The longitudinal cells include longitudinal end cells 108 which are adjacent the respective longitudinal ends 104, 106 of the stent body 102, and longitudinal intermediate cells 110 disposed between the end cells 108. In one embodiment, the end cells 108 at each longitudinal end 104, 106 are identical and the intermediate cells 110 are identical. The stent body 102 may include one or more intermediate cells 110. Three intermediate cells 110 are shown.

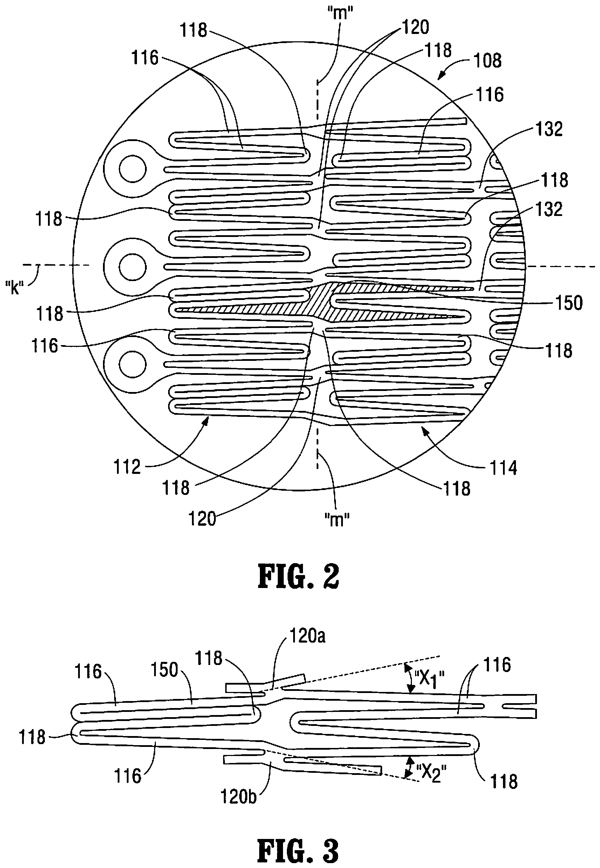

With reference to FIGS. 1 and 2, each end cell 108 includes first and second opposed structural members 112, 114 extending longitudinally and circumferentially with respect to the longitudinal axis "k" and defining an undulating pattern or arrangement. The first and second structural members 112, 114 are generally symmetrically arranged about an axis of symmetry "m" extending between the first and second structural members 112, 114 and orthogonal to the longitudinal axis "k". The first and second structural members 112, 114 may be out of phase with each other (e.g. 90° or 180° out of phase). The symmetrical arrangement of the first and second structural members 112, 114 may or may not be mirror symmetry.

With reference to FIGS. 1 and 2, the first and second structural members 112, 114 include respective individual struts 116 with circumferentially adjacent struts 116 interconnected at apices or nodes 118. The struts 116 may be generally linear and the nodes 118 may be generally arcuate or circular in configuration. Other configurations are also envisioned. The respective lengths of the struts 116 within each end cell 108 may be the same or slightly varied. The struts 116 may taper in width from one end of the strut 116 to the other end, or have different widths along the length of each strut 116 (in, e.g., a non-tapering manner). The thickness of each strut 116 is generally the same or may vary. The spacing between adjacent struts 116 also may vary.

The first and second structural members 112, 114 of the end cells 108 are connected by a plurality of structural end connectors 120, which extend between longitudinally adjacent nodes 118 of the first and second structural members 112, 114. In embodiments, the longitudinally adjacent nodes 118 interconnected by each structural end connector 120 are circumferentially adjacent, e.g., the nodes 118 of the first and second structural members 112, 114 which are closest to each other with respect to the circumference of the stent body 102. In one embodiment, an end connector 120 extends between each alternate pair of longitudinally adjacent nodes 118 of the first and second structural members 112, 114, i.e., every other pair of adjacent nodes 118 is connected by an end connector 120. As best depicted in FIG. 3, a first end connector 120a connecting the first and second structural members 112, 114 extends at a first positive oblique angle "x₁" relative to the longitudinal axis "k" and a second circumferentially adjacent end connector 120b connecting the first and second structural members 112, 114 extends at a second negative oblique angle "x₂" relative to the longitudinal axis "k". The absolute value of the angle of the oblique angles "x₁, x₂" may range from about 5 degrees to about 40 degrees relative to the longitudinal axis "k". In embodiments, the absolute values of the first and second angles "x1", "x2" are substantially the same. Alternatively, the values may be different. In embodiments, the first and second end connectors 120a, 120b may be arranged in alternating manner about the circumference of each end cell 108.

The end connectors 120 increase the radial strength of the stent body 102 and also facilitate deployment of the stent 100 by minimizing the potential of the stent body 102 from "jumping out" of the delivery catheter. In particular, the relative stability and/or strength of the end cells 108, due in part to the construction of the end connectors 120, the increased number of end connectors 120 and the alternating angled arrangement within each end cell 108, ensures that the end cell 108 when deployed from the delivery catheter slowly opens to form almost a funnel shape, and will not release from the delivery catheter until, e.g., the entire adjacent intermediate cell 110 is exposed.

The first and second end connectors 120a, 120b define a closed cell segment 150 of each end cell 108. The closed cell segment 150 is depicted in FIG. 2 as the cross-hatched area and in the isolated view of FIG. 3. The cell segment 150 is inclusive of the struts 116, the nodes 118 of the respective first and second structural members 112, 114, and the first and second end connectors 120a, 120b. Each end cell 108 may have from 4 to 20 closed cell segments depending on the diameter of the stent body. The cell segments will be discussed in greater detail hereinbelow.

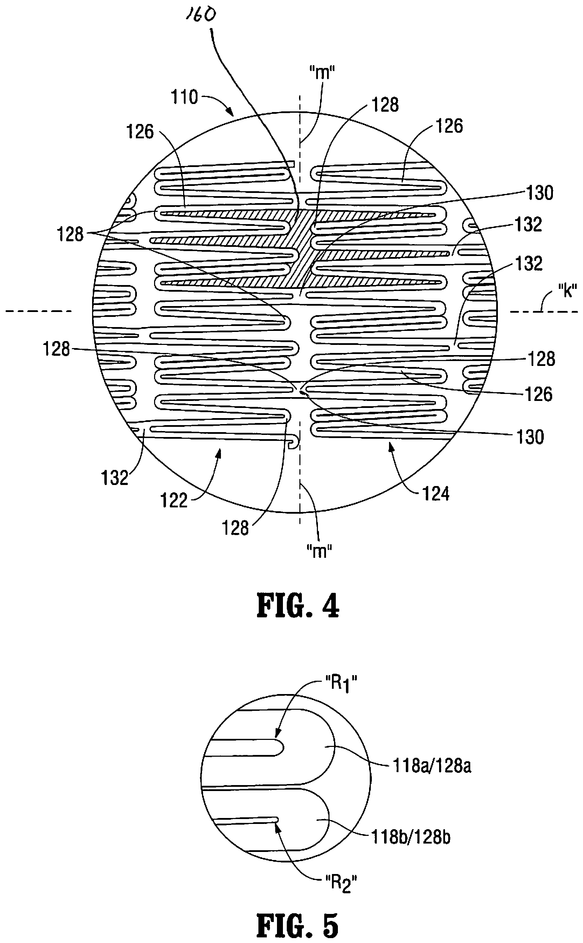

Referring now to FIG. 4, in conjunction with FIG. 1, the intermediate cells 110 also include first and second structural members 122, 124 arranged in a similar manner to the first and second structural members 112, 114 of the end cells 108. The first and second structural members 122, 124 include respective individual struts 126 with circumferentially adjacent struts 126 interconnected at the apices or nodes 128. The number and/or configuration of struts 126 and nodes 128 may be the same for the intermediate and the end cells 108, 110.

The first and second structural members 122, 124 of each intermediate cell 110 are interconnected by a plurality of structural intermediate connectors 130 which extend between longitudinally adjacent nodes 128 of the first and second structural members 122, 124. In one embodiment, the intermediate connectors 130 are in general parallel relation with the longitudinal axis "k" of the stent body 102 when in the unexpanded condition of FIG. 1. In the alternative, the intermediate connectors 130 may be in oblique relation with the longitudinal axis "k". In embodiments, the longitudinally adjacent nodes 128 interconnected by each structural intermediate connector 130 are circumferentially adjacent or longitudinally aligned.

Each intermediate cell 110 has fewer intermediate connectors 130 than the number of end connectors 120 within the end cells 108. In one embodiment, each intermediate cell 110 includes "x" number of intermediate connectors 130 and each end cell includes "x +1" number of end connectors 120. In embodiments, the end cells 108 include twice or "2x" the number of end connectors 120 than the number of intermediate connectors 130 of the intermediate cells 108. In one embodiment, an intermediate connector 130 extends between each fourth pair of longitudinally adjacent nodes 128 of the first and second structural members 122, 124 of the intermediate cells 110. In embodiments, the end cells 108 include from about six to twelve end connectors 120 and the intermediate cells include from three to six intermediate connectors 130. In embodiments, the end cells 108 include six end connectors 120 and the intermediate cells 110 include three intermediate connectors 130.

The intermediate connectors 130 of the intermediate cells 110 define closed cell segments 160 depicted as the cross hatched area in FIG. 4. Each closed cell segment 160 is inclusive of the struts 126, nodes 128 of the respective first and second structural members 122, 124, and the first and second intermediate connectors 130. Each intermediate cell 110 may have from 2 to 10 closed cell segments 160 depending on the diameter of the stent body 102, and, in some embodiments, have half the number of closed cell segments relative to the end cells 108. The closed segments 160 will be discussed in greater detail hereinbelow.

With reference again to FIGS. 1 and 2, the end cells 108 are connected to the adjacent intermediate cells 110 via a plurality of cell connectors 132. Adjacent intermediate cells 110 are also connected to each other via a plurality of cell connectors 132. (see also FIG. 4). In embodiments, the cell connectors 132 may be substantially similar in configuration to the intermediate connectors 130 of the intermediate cells 110, and arranged in general parallel relation to the longitudinal axis "k' of the stent body 102 when in the unexpanded initial condition. In the alternative, the cell connector 132 may be in oblique relation to the longitudinal axis "k".

With reference to FIG. 5, in conjunction with FIG. 1, the stent body 102 may include at least two different configurations for the nodes 118, 128. The first node types 118a, 128a are distributed throughout the stent body 102 including the end and intermediate cells 108, 110. In one embodiment, the first node types 118a, 128a includes a generally semi-circular arcuate segment having a radius of curvature "R₁". The radius of curvature "R₁" is defined as the distance of the circular arc which best approximates the curve at that point, and is measured along an inside edge of the node. The radius of curvature "R₁" may be constant. In embodiments, the radius of curvature "R₁" has a non-constant radius or compound radius. The non-constant or compound radius advantageously lowers peak strain on the first nodes 118a, 128a by distributing strain experienced by the stent body 102, thereby improving the fatigue life of the particular node and adjacent struts, and, consequently improving the overall fatigue life of the stent 100. Further details of the first node types 118a, 128a may be ascertained by reference to commonly assigned U.S. Patent Application Serial Nos.: 13/834,840, filed March 15, 2103 and 13/834,713, filed March 15, 2013.

The second node types 118b, 128b may be either devoid of an internal curvature or have a small radius of curvature, each being represented as "R₂", which is substantially less than the radius curvature "R₁" of the first nodes 118a, 128a. The ability to incorporate the second node types 118b, 128b within the stent body 102 is due to aforementioned strain distributions within the stent body 102 provided by the first node types 118a, 128a. Thus, it is envisioned that the second node types 118b, 128b would be subjected to less strain. The particular dimensioning of the radii of curvature of the first and second node types may be produced during manufacture of the stent 102.

Referring again to FIG. 1, the stent body may include a plurality of eyelets 134 at one of both of the longitudinal ends of the body. The eyelets 134 extend from respective nodes or peaks 118 of the end cells 108. The eyelets 134 may be filled with a radiopaque material to facilitate visualization of the stent body 102 during and subsequent to implantation.

The stent 100 may be fabricated from any suitable shape memory or super-elastic material such as nickel titanium (e.g., Nitinol). In embodiments, the super-elastic material is treated to cause the stent body 102 to expand to its Austenitic memory state when released from a constrained condition to assume a predetermined deployed or expanded diameter. The stent 100 may come in a variety of sizes and lengths. In a venous application, the stent may be 10 millimeters (mm), 12mm, 14 mm, 16mm, 18mm or 20mm in diameter, and 40mm to 150mm in length. Other diameters and lengths are also envisioned.

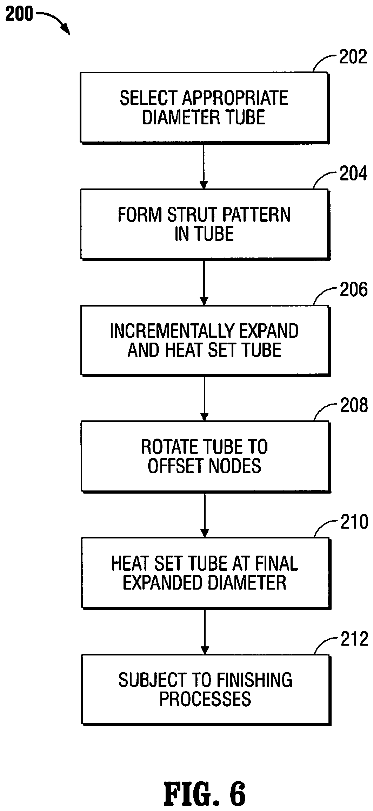

FIG. 6 is a flow chart illustrating a process for manufacture of the stent 100. In accordance this process of manufacture 200, a nitinol tube having a defined diameter is selected. (Step 202). For a venous application, the stent 100 may require a greater wall thickness relative to arterial stents, e.g., 0.4572 mm (.018 inches) for the 10, 12 and 14 mm stents and 0.7112 mm (.028 inches) inches for the 16, 18 and 20mm stents. The tube is then positioned with respect to the laser. The laser, which is programmed to provide the strut and cell pattern of the stent body 102 described hereinabove, is activated to form the stent pattern inclusive of the longitudinal cells, structural members, struts, slots and nodes. (Step 204) When forming the first node type 118a, 128a, multiple passes of the laser may be required to create the non-uniform or compound radius of curvature "R₁". However, due to the constrained design of the second node type 118b, 128b, only a single pass of the laser is needed to form the slot between the adjacent struts, which extends to the nodes 118a, 128a. As discussed hereinabove, the radius of curvature "R₁" of the first node type 118a, 128a is designed to distribute strain and thereby increase fatigue life of the stent, and eliminates the need for defined curvature within the second node type 118b, 128b. Consequently, this constrained geometry of the second node type 118b, 128b, requiring only a single pass of the laser, reduces the number of burrs and/or defects presented in this region during processing, thus facilitating manufacture and reducing cycle time to produce the stent 100.

The cut tube is then subjected to a shape-setting process in which the cut tube is expanded on a mandrel and then heated. (Step 206) Multiple incremental expansions and heating cycles can be used to shape-set the stent body 102 to a desired expanded diameter. It is envisioned that the final expanded diameter may be equal to the desired deployed diameter of the stent body. The stent body 102 may be axially restrained such that the length of stent 100 does not change during expansion.

The stent body 102 is then subjected to a twisting step, which involves imparting a slight helical twist to the stent body 102. (Step 208) One objective of the helical twist is to offset longitudinally adjacent nodes to minimize the potential of these nodes contacting each other when the stent body 102 is implanted in the subject and subjected to physical stress or strain during, e.g., movement of the subject.

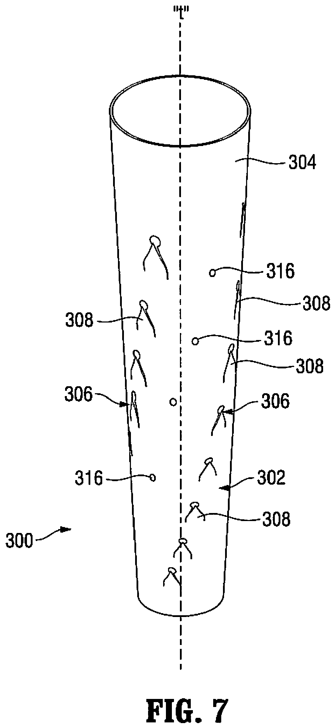

With reference now to FIGS. 7-9, the final expansion process includes a twisting mandrel 300 upon which the laser cut stent body 102 is positioned. The mandrel 300 includes a substantially rigid cylindrical member 302 fabricated from stainless steel or a rigid polymer. The mandrel 300 includes an outer wall 304 arranged about a central axis "t" and having multiple of series 306 of projections 308 extending from the outer wall 304. Each series 306 of projections 308 are arranged in a helical pattern about the outer wall 304 and relative to the central axis "t" with adjacent series 306 being circumferentially spaced from each other a predetermined distance. In embodiments, each adjacent series 306 is circumferentially spaced the same distance. Each projection 308 is generally pyramidal or triangular shape in shape having opposed sides 310 which converge and terminate at an apex 312. (See FIG. 7) The sides 310 are elevated relative to the outer wall of the mandrel, e.g., arranged at an angle with respect to the central axis "t" such that displacement of the sides 310 is greater approaching the apex 312. The projections 308 may be cut, e.g., with a laser, into the cylindrical member 302 (if a metallic material) or formed during injection molding (if a polymeric material).

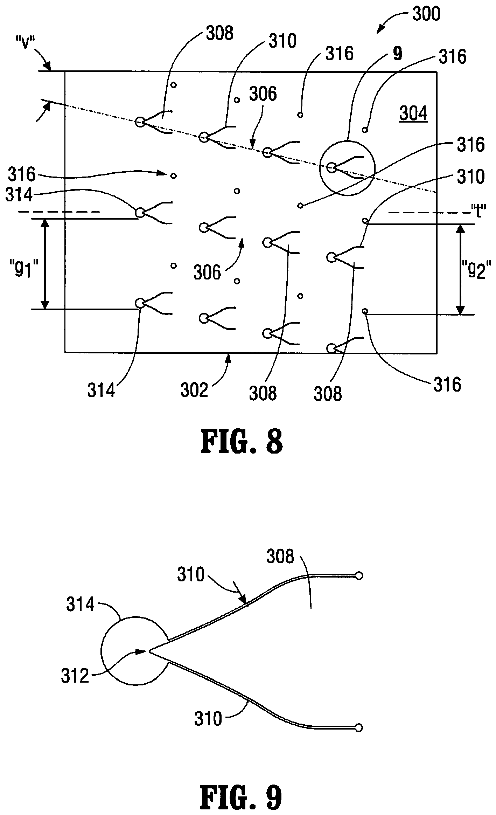

As best depicted In FIG. 8, the helix angle "j" about which the series 306 of the projections 308 are arranged may be from about 8 degrees to about 16 degrees, and, may be about 12 degrees. The helix angle "j" is sufficient to offset the longitudinal adjacent nodes of the stent body 102. Adjacent each apex 312 of the projections 308 is an enlarged aperture 314. The apertures 314 may assist the clinician in positioning and/or guiding the nodes 118, 128 of the stent body 102 about the apex 312 of the projections 308. The adjacent apertures 314 are circumferentially spaced a distance "g1".

The stent body 102 also includes a plurality of series of smaller guide apertures 316. The smaller guide apertures 316 are longitudinally adjacent the base of each projection 308 and disposed between circumferentially adjacent projections 308. The guide apertures 316 are arranged about substantially the same helix angle as the helix angle "j" of the series 306 of the projections 308. The guide apertures 316 are also spaced about the circumference a distance "g2" which is substantially the same as the distance "g1" between the enlarged apertures 314. The guide apertures 316 assist in confirming that the stent body 102 is properly positioned about the mandrel 300 as will be discussed.

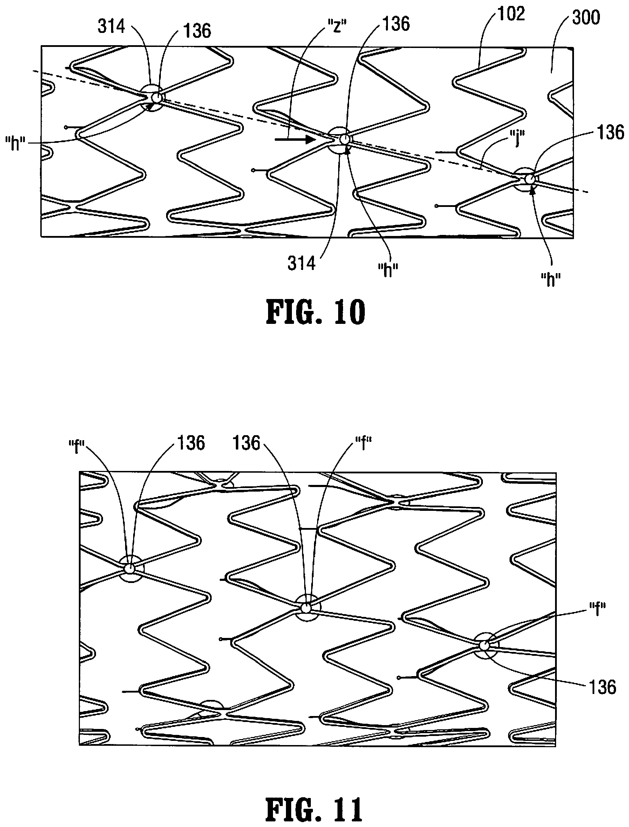

Referring now to FIGS. 10-11, once the stent body 102 is prepared for final expansion and twisting, the stent body 102 is positioned on the mandrel 300 by advancing the stent body 102 in the direction of the projections 308 as indicated by directional arrow "z". A select series of connected nodes 136 (the connected nodes 136 may be any nodes interconnected by an end, intermediate or cell connector 120, 130, 132) are aligned with a series 306 of the projections 308 arranged at the defined helix angle "j". During this positioning, one or more of the longitudinal ends 104, 106 of the stent body 102 will be rotated or twisted to ensure the connected nodes are disposed over the projections 308. The enlarged apertures 314 may assist in aligning the connected nodes. The connected nodes 136 are then placed over the projections 308 whereby the projections 308 engage and hook the connected nodes 136 and/or struts of the stent body 102. The enlarged apertures 314 may receive at least a portion of the connected nodes 136 to facilitate engagement with the connected nodes 136. The sides 310 of the projections 308 may also follow the profile of the adjacent struts and engage the struts to further retain the stent body 102 relative to the projections 308. The regions "h" of FIG. 10 schematically depict the positioning of the connected nodes about the projections 308.

In one embodiment, every other circumferentially adjacent connected node series is positioned about the series of projections 308. In accordance with this embodiment, the smaller guide apertures 316 are utilized to ensure that these connected nodes 136 are in alignment with the series of guide apertures 316 thereby providing visual confirmation that the stent body 102 is properly aligned with respect to the projections 308 and the mandrel 300, i.e., the positioning of these connected nodes 136 should be in general alignment with the guide apertures 316 due to the symmetry of positioning of at least one of the projections 308, enlarged apertures 314 and the guide apertures 316. The regions "f" of FIG. 11 schematically depict the positioning of the connected nodes adjacent the guide apertures 316.

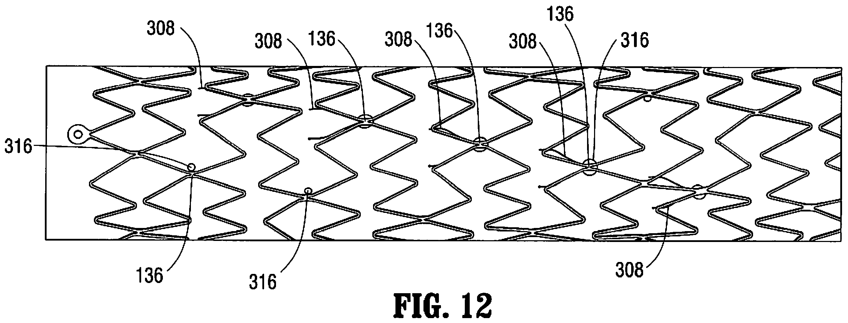

FIG. 12 illustrates the stent body 102 positioned on the mandrel 300. One series of connected nodes 136 is depicted engaged with the series of projections 308 and the next circumferentially adjacent series of connected nodes 136 are aligned with the guide apertures 316 and not connected to any projections 308. This series of connected nodes 136 is generally aligned with the guide apertures 316.

With reference again to FIG. 6, the manufacturing process is continued by subjecting the stent body 102 mounted about the mandrel 300 is to heat treatment to heat set the final expanded and twisted condition of the stent body 102. (STEP 210) The stent body 102 may be subjected to a finishing and/or coating process (STEP 212) including electro-polishing and/or application of a coating.

The aforedescribed process for manufacture of the stent 200 may be modified. For example, it is envisioned that Step 210 may or not expand the stent tube to its final diameter. Furthermore, Step 206 may be eliminated with expansion of the stent tube to its final diameter occurring in Step 210.

The use of the stent 100 will now be discussed. The stent 100 is mounted on a delivery catheter. As is conventionally known in the art, the stent 100 may be confined in the initial condition of FIG. 1 on the delivery catheter by a retractable sheath. The delivery catheter can be used to advance the stent 100 to a deployment site (e.g., a constricted region of a vessel). At the deployment site, the sheath is retracted thereby releasing the stent 100. Once released, the stent 100 self-expands to the deployed diameter or expanded condition thereof.

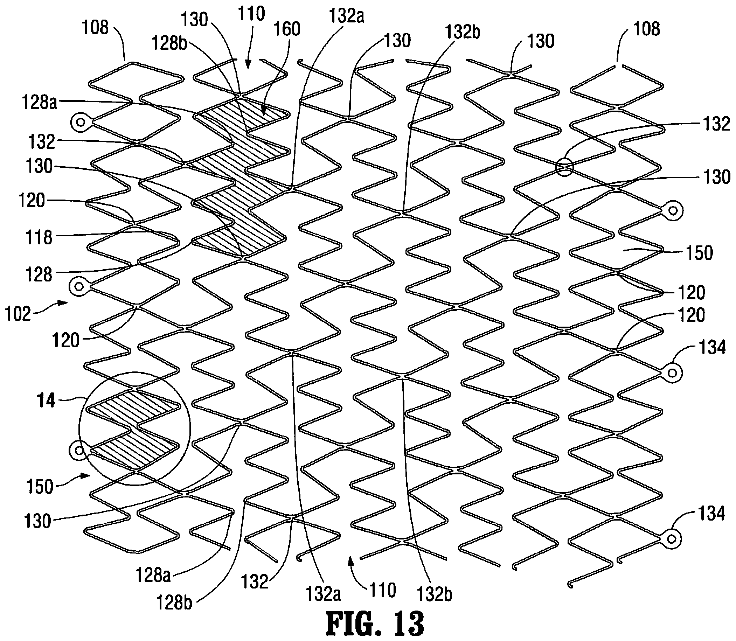

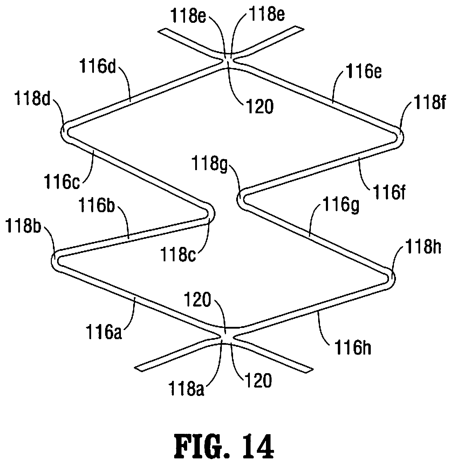

FIG. 13 illustrates the stent 100 in the final expanded condition. In FIG. 13, the stent 100 is unrolled and depicted in plan view as a flat sheet. The expanded stent 100 includes the end cells 108 and the intermediate cells 110. The end cells 108 each include a plurality of the closed cell segments 150. As depicted in FIG. 14, each cell segment 150 is shown including: 1) a first strut 116a that extends from a first node 118/end connector 120 or valley 118a to a second node or peak 118b; 2) a second strut 116b that extends from the second peak 118b to a third node or valley 118c; 3) a third strut 116c that extends from the third valley 118c to a fourth node or peak 118d; 4) a fourth strut 116d that extends from the fourth peak 118d to a fifth node 118e/end connector 120 or valley 118e; 5) a fifth strut 116e that extends from the fifth valley 118e to a sixth node or peak 118f ; 6) a sixth strut 116f that extends from the sixth peak 118f to a seventh node or valley 116g; 7) a seventh strut 116g that extends from the seventh valley 118g to an eighth node or peak 118h; and 8) an eighth strut 116h that extends from the eighth peak 118h to the first valley 118a to complete the closed cell segment 150. The end cell 108 may include any number of closed cell segments, e.g., having six to twelve closed cell segments 150, and in embodiments, six cell segments 150.

With reference again to FIG. 13, each intermediate cell 110 follows the general pattern of the end cell 108. The pattern will not be repeated herein for the sake of clarity; however, each intermediate cell includes a cell segment 160 (shown in hash marks) having sixteen struts, eight peaks and eight valleys two of which are defined by the intermediate connectors 130. Each intermediate cell 110 may have from three to six closed cell segments 160. In embodiments, each intermediate cell 110 includes three cell segments 160. In some embodiments, the intermediate cells 110 include one-half the number of closed cell segments 160 compared to the number of cell segments 150 of the end cells 108.

In the expanded condition of the stent body depicted in FIG. 13, longitudinally adjacent nodes 118, 128 of the longitudinally adjacent end and intermediate cells 108, 110 are in general longitudinal alignment. However, longitudinally adjacent nodes 128 (e.g., 128a, 128b in FIG. 13) within each intermediate cell 110 may be circumferentially displaced or misaligned. Circumferential displacement is the displacement occurred along the circumference or perimeter of the stent body 102. In addition, circumferentially adjacent cell connectors 132a, 132b (e.g., the cell connectors 132a, 132b closest with respect to the circumference of the stent body 102) extending between adjacent intermediate cells 110 are circumferentially displaced. This feature is provided by the heat set process with the mandrel. The offset nodes 128a, 128b (FIG. 13) within the intermediate cells 110 permits flexure of the stent body 102 when in the vasculature of the subject with minimal contact or "node knocking" of the adjacent nodes. This improves the fatigue life of the stent 100.

The configuration of the stent body 102 including the end cells 108 and the intermediate cells 110 provide substantial advantages when placed in, e.g., the venous system of the subject. The increased presence or number of the connectors 120 of the end cells 108 provides stability and radial strength to the longitudinal ends 104, 106 of the stent body 102. This increased stability of the end cells 108 advantageously prevents jumping or premature "flowering" of the end cells 108 upon deployment from the delivery catheter. Thus, control over the deployment of the stent 100 is facilitated. In addition, at least the end cell 108 of the stent 100 may be more readily resheathed or returned within the lumen of the delivery catheter in the event the stent 100 needs to be repositioned relative to the operative site. The relative reduced number of connectors 130 within the intermediate cells 110 provides the requisite flexibility required for implantation within the venous site, and accommodates for movement of the limb, pelvis, hip, etc.

Numerous other modifications are possible with the stent 100. For example the stent 100 may be lined with either an inner or outer sleeve (such as polyester fabric or ePTFE) to facilitate tissue growth. Also, at least a portion of stent 100 may be coated with radiopaque coatings such as platinum, gold, tungsten or tantalum. In addition to materials previously discussed, stent 100 may be formed of other materials, including, without limitation, MP35N, tantalum, platinum, gold, Elgiloy and Phynox.

While an envisioned use for the features disclosed in the accompanying figures relates to that of a self-expanding stent, the features also have benefits when used with non-self-expanding stents (e.g., balloon expandable stents made of a material such as stainless steel).

## Claims

1. A medical stent (100) comprising:
a stent body (102) defining a longitudinal axis "K" and opposed longitudinal ends (104,106), the stent body (102) adapted to expand from an initial condition to an expanded condition, the stent body (102) including a plurality of longitudinal cells, the longitudinal cells including a first end cell (108) and a second end cell (108) opposite each other and at least one intermediate cell (110) disposed between the first end cell (108) and the second end cell (108);
each longitudinal cell having first and second structural members (112, 114,122,124) extending in an undulating pattern about the longitudinal axis "K";
x number of intermediate connectors (130) interconnecting the first and second structural members (122,124) of the at least one intermediate cell (110); and
at least 2x the number of end connectors (120) interconnect the first and second structural members (112,114) of each of the first end cell (108), and the second end cell (108),
wherein the end connectors (120) comprise a first end connector (120a) and a second end connector (120b) interconnecting the first and second structural members (112,114) of the first end cell (108) and the second end cell (108), and
wherein in the initial condition of the stent body (102), the first end connector (120a) is arranged at a positive first oblique angle relative to the longitudinal axis "K", and the second end connector (120b) is arranged at a negative second oblique angle relative to the longitudinal axis "K".

2. The stent (100) according to claim 1 wherein the intermediate connectors (130) are each arranged in general parallel relation with the longitudinal axis "K".

3. The stent (100) according to claim 1 further including cell connectors (132) for interconnecting adjacent longitudinal cells.

4. The stent (100) according to claim 3 wherein x number of cell connectors (132) interconnect each of the first end cell (108) and the second end cell (108) to the at least one intermediate cell (110).

5. The stent (100) according to claim 4 wherein adjacent intermediate cells (110) are interconnected by x number of cell connectors (132).

6. The stent (100) according to claim 1 wherein the first and second structural members (112,114,122,124) of each cell (108,110) each include a plurality of struts (116,126) with adjacent struts (116,126) being interconnected by a node (118,128).

7. The stent (100) according to claim 6 wherein the end connectors (120) and intermediate connectors (130) are dimensioned to interconnect longitudinally adjacent nodes (118) of the first and second structural members (112,114) of the first end cell (108) and the at least one intermediate cell (110), wherein at least some of the longitudinally adjacent nodes (128) of the at least one intermediate cell (108) are circumferentially offset relative to each other when the stent body (102) is in the expanded condition.

8. The stent according to claim 7 further including cell connectors (132) dimensioned to interconnect longitudinally adjacent nodes (118,128) of adjacent longitudinal cells, wherein at least some of the cell connectors (132) interconnecting adjacent longitudinal cells are circumferentially offset relative to each other when the stent body (108) is in the expanded condition.

9. The stent according to claim 6 wherein the nodes (118,128) include a first node type (118a,128a) and second node type (118b, 128b), the first node type (118sa,128a) having an internal curvature defining at least a first radius of curvature (Ri), and the second node type (118b,128b) having an internal curvature defining a second radius of curvature (R₂) less than the first radius of curvature (R₁).

## Patentansprüche

1. Medizinischer Stent (100), Folgendes umfassend:
einen Stentkörper (102), der eine Längsachse "K" und gegenüberliegende Längsenden (104, 106) definiert, wobei der Stentkörper (102) angepasst ist, um sich von einem Anfangszustand in einen erweiterten Zustand zu erweitern, wobei der Stentkörper (102) mehrere Längszellen enthält, wobei die Längszellen eine erste Endzelle (108) und eine zweite Endzelle (108) einander gegenüberliegend enthalten und mindestens eine Intermediärzelle (110), die zwischen der ersten Endzelle (108) und der zweiten Endzelle (108) angeordnet ist;
wobei jede Längszelle erste und zweite Strukturelemente (112, 114, 122, 124) aufweist, die sich in einem wellenförmigen Muster um die Längsachse "K" erstrecken;
eine x-Anzahl von Zwischenverbindern (130), die das erste und das zweite Strukturelement (122, 124) der mindestens einen Intermediärzelle (110) verbinden; und
mindestens 2x die Anzahl der Endverbinder (120), die das erste und das zweite Strukturelement (112, 114) von jeder ersten Endzelle (108) und jeder zweiten Endzelle (108) verbinden, wobei die Endverbinder (120) einen ersten Endverbinder (120a) und einen zweiten Endverbinder (120b) umfassen, die das erste und zweite Strukturelement (112, 114) der ersten Endzelle (108) und der zweiten Endzelle (108) verbinden, und wobei im Anfangszustand des Stentkörpers (102) der erste Endverbinder (120a) in einem positiven ersten Schrägwinkel in Bezug auf die Längsachse "K" angeordnet ist, und der zweite Endverbinder (120b) in einem negativen zweiten Schrägwinkel in Bezug auf die Längsachse "K" angeordnet ist.

2. Stent (100) nach Anspruch 1, wobei die Zwischenverbinder (130) jeweils im Wesentlichen parallel zur Längsachse "K" angeordnet sind.

3. Stent (100) nach Anspruch 1 ferner enthaltend Zellverbinder (132) zum Verbinden benachbarter Längszellen.

4. Stent (100) nach Anspruch 3, wobei die x-Anzahl von Zellverbindern (132) jede erste Endzelle (108) und jede zweite Endzelle (108) mit der mindestens einen Intermediärzelle (110) verbinden.

5. Stent (100) nach Anspruch 4, wobei benachbarte Intermediärzellen (110) durch die x-Anzahl von Zellverbindern (132) miteinander verbunden sind.

6. Stent (100) nach Anspruch 1, wobei das erste und zweite Strukturelement (112, 114, 122, 124) jeder Zelle (108, 110) jeweils mehrere Streben (116, 126) beinhalten, wobei benachbarte Streben (116, 126) durch einen Knoten (118, 128) verbunden sind.

7. Stent (100) nach Anspruch 6, wobei die Endverbinder (120) und die Zwischenverbinder (130) bemessen sind, um längs benachbarte Knoten (118) der ersten und zweiten Strukturelemente (112, 114) der ersten Endzelle (108) und der mindestens einen Intermediärzelle (110) miteinander zu verbinden, wobei mindestens einige der längs benachbarten Knoten (128) der mindestens einen Intermediärzelle (108) in Umfangsrichtung zueinander versetzt sind, wenn sich der Stentkörper (102) im erweiterten Zustand befindet.

8. Stent nach Anspruch 7 ferner enthaltend Zellverbinder (132), bemessen, um längs benachbarte Knoten (118, 128) benachbarter Längszellen zu verbinden, wobei mindestens einige der Zellverbinder (132), die benachbarte Längszellen verbinden, in Umfangsrichtung zueinander versetzt sind, wenn sich der Stentkörper (108) im erweiterten Zustand befindet.

9. Stent nach Anspruch 6, wobei die Knoten (118, 128) einen ersten Knotentyp (118a, 128a) und einen zweiten Knotentyp (118b, 128b) enthalten, wobei der erste Knotentyp (118a, 128a) eine innere Krümmung aufweist, die mindestens einen ersten Krümmungsradius (R₁) definiert, und wobei der zweite Knotentyp (118b, 128b) eine innere Krümmung aufweist, die einen zweiten Krümmungsradius (R₂) definiert, der kleiner als der erste Krümmungsradius (R₁) ist.

## Revendications

1. Stent médical (100) comprenant :
un corps de stent (102) définissant un axe longitudinal « K » et des extrémités longitudinales opposées (104, 106), le corps de stent (102) étant adapté pour se dilater d'un état initial à un état dilaté, le corps de stent (102) comprenant une pluralité de cellules longitudinales, les cellules longitudinales comprenant une première cellule d'extrémité (108) et une seconde cellule d'extrémité (108) opposées et au moins une cellule intermédiaire (110) disposée entre la première cellule d'extrémité (108) et la seconde cellule d'extrémité (108) ;
chaque cellule longitudinale comportant des premier et second éléments structurels (112, 114, 122, 124) s'étendant selon un motif ondulé autour de l'axe longitudinal « K » ;
x nombre de connecteurs intermédiaires (130) reliant les premier et second éléments structurels (122, 124) d'au moins une cellule intermédiaire (110) ; et
au moins 2 fois le nombre de connecteurs d'extrémité (120) relient les premier et second éléments structurels (112, 114) de chacune de la première cellule d'extrémité (108), et de la seconde cellule d'extrémité (108), dans lequel les connecteurs d'extrémité (120) comprennent un premier connecteur d'extrémité (120a) et un second connecteur d'extrémité (120b) reliant les premier et second éléments structurels (112, 114) de la première cellule d'extrémité (108) et de la seconde cellule d'extrémité (108), et dans lequel, à l'état initial du corps de stent (102), le premier connecteur d'extrémité (120a) est disposé au niveau d'un premier angle oblique positif par rapport à l'axe longitudinal « K », et le second connecteur d'extrémité (120b) est disposé au niveau d'un second angle oblique négatif par rapport à l'axe longitudinal « K ».

2. Stent (100) selon la revendication 1, dans lequel les connecteurs intermédiaires (130) sont disposés chacun en relation parallèle générale avec l'axe longitudinal « K ».

3. Stent (100) selon la revendication 1, comprenant en outre des connecteurs de cellules (132) destinés à relier des cellules longitudinales adjacentes.

4. Stent (100) selon la revendication 3, dans lequel x nombre de connecteurs de cellules (132) relient chacune des première (108) et seconde cellules d'extrémité (108) à l'au moins une cellule intermédiaire (110).

5. Stent (100) selon la revendication 4, dans lequel des cellules intermédiaires adjacentes (110) sont reliées par un nombre x de connecteurs de cellules (132).

6. Stent (100) selon la revendication 1, dans lequel les premier et second éléments structurels (112, 114, 122, 124) de chaque cellule (108, 110) comprennent chacun une pluralité d'entretoises (116, 126) comprenant des entretoises adjacentes (116, 126) reliées par un nœud (118, 128).

7. Stent (100) selon la revendication 6, dans lequel les connecteurs d'extrémité (120) et les connecteurs intermédiaires (130) sont dimensionnés pour relier des noeuds longitudinaux adjacents (118) des premier et second éléments structurels (112, 114) de la première cellule d'extrémité (108) et de l'au moins une cellule intermédiaire (110), dans lequel au moins certains des noeuds longitudinaux adjacents (128) de l'au moins une cellule intermédiaire (108) sont décalés circonférentiellement les uns des autres lorsque le corps de stent (102) est à l'état dilaté.

8. Stent selon la revendication 7, comprenant en outre des connecteurs de cellules (132) dimensionnés pour relier des noeuds longitudinalement adjacents (118, 128) de cellules longitudinales adjacentes, dans lequel au moins certains des connecteurs de cellules (132) reliant des cellules longitudinales adjacentes sont décalés circonférentiellement les uns des autres lorsque le corps de stent (108) est à l'état dilaté.

9. Stent selon la revendication 6, dans lequel les noeuds (118, 128) comprennent un premier type de nœud (118a, 128a) et un second type de nœud (118b, 128b), le premier type de nœud (118sa, 128a) comportant une courbure interne définissant au moins un premier rayon de courbure (R₁), et le second type de nœud (118b, 128b) comportant une courbure interne définissant un second rayon de courbure (R₂) inférieur au premier rayon de courbure (R₁).
